Europäisches Patentamt

European Patent Office

Office européen des brevets

(19)

(11) Numéro de publication: **0 149 936**
**A2**

## (12) DEMANDE DE BREVET EUROPEEN

(21) Numéro de dépôt: **84402602.1**

(22) Date de dépôt: **14.12.84**

(51) Int. Cl.⁴: **C 12 N 1/20**
C 12 N 9/86, C 12 N 15/00
//(C12N1/20, C12R1:19)

(30) Priorité: **14.12.83 FR 8320022**

(43) Date de publication de la demande:
**31.07.85 Bulletin 85/31**

(84) Etats contractants désignés:
**AT BE CH DE GB IT LI LU NL SE**

(71) Demandeur: **INSTITUT MERIEUX Société anonyme dite:**
**17, rue Bourgelat**
**F-69002 LYON(FR)**

(72) Inventeur: **Fognini-Lefebvre, Nicole**
**69, boulevard Eugène Réguillon**
**F-69100 Villeurbanne(FR)**

(72) Inventeur: **Lazzaroni, Jean-Claude**
**30, route de Genève Saint Maurice de Beynost**
**F-01700 Miribel(FR)**

(72) Inventeur: **Portalier, Raymond**
**8, avenue Jean-Jaurès**
**F-69007 Lyon(FR)**

(74) Mandataire: **Nony, Michel**
**Cabinet Nony 29, rue Cambacérès**
**F-75008 Paris(FR)**

(54) Nouveaux mutants d'escherichia coli, capables d'excréter dans le milieu extracellulaire des protéines périplasmiques, leur obtention et leur application à la préparation de bêta-lactamases ou de protéines hybrides de bêta-lactamases.

(57) Mutants d'*Escherichia coli*, capables d'excréter dans le milieu extracellulaire des proteines périplasmiques, caractérisés par le fait qu'ils sont issus d'une souche parente capable de synthétiser une bêta-lactamase ou une protéine hybride comportant une séquence amino-terminale analogue à celle de la bêta-lactamase, ladite souche parente n'excrétant pas les protéines périplasmiques y compris la bêta-lactamase ou ladite protéine hybride, ou n'en excrétant pas plus de 5 % à 30°C, que lesdits mutants sont porteurs d'une mutation contransductible à plus de 50% avec le marqueur *gal*, qu'ils sont, comme la souche parente, non sensibles ou peu sensibles à un ou plusieurs des inhibiteurs de croissance choisis parmi l'acide cholique, le chloramphénicol et l'EDTA, qu'ils sont sensibles, comme la souche parente, à la colicine El, que, comme la souche parente, ils n'excrètent pas la phosphatase alcaline ou n'en excrètent pas plus de 20 % à 30°C, et qu'ils excrètant au moins 50 % de la bêta-lactamase (ou de ladite protéine hybride) qu'ils produisent à 37°C; ainsi que leur obtention.

Application notamment à la production de bêta-lactamases.

Nouveaux mutants d'Escherichia coli, capables d'excréter dans le milieu extracellulaire des protéines périplasmiques, leur obtention et leur application à la préparation de bêta-lactamases ou de protéines hybrides de bêta-lactamases.

La présente invention a pour objet de nouveaux mutants d'Escherichia coli, capables d'excréter dans le milieu extracellulaire des protéines périplasmiques, leur obtention et leur application notamment à la production de bêta-lactamases ou de protéines hybrides dérivées d'une bêta-lactamase.

On sait que l'enveloppe cellulaire des bactéries Escherichia coli est constituée de deux membranes : la membrane interne (ou cytoplasmique) et la membrane externe, qui délimitent ainsi le cytoplasme et l'espace périplasmique.

Cet espace périplasmique représente généralement 5 à 10 % du volume cellulaire total, cette proportion pouvant toutefois être plus importante selon les conditions de culture.

Une description de l'enveloppe cellulaire, avec les membranes interne et externe, des bactéries GRAM négatives telles qu'Escherichia coli est donnée par exemple par : - COSTERTON J.W. (1975), J. Antimicrob. Chemother. 1 : 363-377.

Contrairement à la membrane cytoplasmique, la membrane externe contient un nombre limité d'espèces protéiques, parmi lesquelles, certaines, appelées majoritaires, sont présentes à des concentrations très élevées : ce sont notamment les protéines OmpF, OmpC, OmpA et PhoE ; voir par exemple :
- OSBORN M.J. and H.C.P. WU (1980), Ann. Rev. 34 : 369-422 ;
- REEVES P. (1979), in Bacterial Outer Membranes (M. INOUYE ed.), John Wiley and Sons Inc. New-York, 255-291 ;
- LUGTENBERG B. (1981), TIBS 6 : 262-266.

Les nomenclatures utilisées ici sont conformes à celle de OSBORN M.J. and H.C.P. WU pour les protéines, op. cit. et à celle de BACHMAN B.J. and K.B. LOW (1980), Microbiol. Rev. 44 : 1-56 pour les marqueurs génétiques.

Le périplasme renferme notamment certaines protéines fixatrices associées au transport actif de certains composés et certaines enzymes, de type dégradatif pour la plupart, appelées enzymes périplasmiques, parmi lesquelles on peut citer la phosphatase alcaline, la 5'-nucléotidase, la phosphodiestérase cyclique, la ribonucléase I, la désoxyribonucléase I, la bêta-lactamase et certaines enzymes intervenant dans la dégradation des nucléosides.

Les enzymes périplasmiques, tout comme les protéines présentes dans la membrane externe, sont synthétisées dans le cytoplasme et doivent, par conséquent, être exportées à travers la membrane cytoplasmique.

On sait que les protéines périplasmiques peuvent être libérées dans le milieu extracellulaire par des traitements qui n'altèrent pas l'intégrité de la membrane cytoplasmique tels que le choc osmotique, ou la

transformation des cellules bactériennes en sphéroplastes ; voir par exemple :

- HEPPEL L.A. (1971), in Structure and Function of Biological Membranes (L.I. ROTHFIELD ed.), Academic Press Inc. New-York, 233-247.

La localisation précise et le rôle physiologique exact des enzymes périplasmiques ne sont pas connus ; ces enzymes pourraient intervenir dans la dégradation de composés toxiques ou anormaux en catalysant des réactions qui seraient néfastes pour la cellule si elles étaient effectuées dans le cytoplasme.

L'obtention à l'échelle industrielle de certaines protéines fabriquées naturellement ou artificiellement par des bactéries, pose des problèmes difficiles d'isolement de la protéine désirée parmi les nombreuses autres protéines présentes dans l'espace périplasmique et dans le cytoplasme.

Pour cette raison, il serait intéressant d'obtenir des bactéries modifiées capables d'excréter de manière sélective dans le milieu extracellulaire une protéine désirée ou un nombre limité de protéines.

Des mutants d'_Escherichia coli_ excrétant dans le milieu extracellulaire certaines enzymes périplasmiques, parmi lesquelles la phosphatase alcaline, ont été décrits par :

- LAZZARONI J.C. and R. PORTALIER (1981), J. Bacteriol. 143 : 1351-1358.

Ces mutants excréteurs de phosphatase alcaline sont sensibles aux inhibiteurs de croissance tels que l'EDTA et l'acide cholique et sont résistants à la colicine E1.

La présente invention a pour objet de nouveaux mutants d'_Escherichia coli_ qui sont capables d'excréter dans le milieu extracellulaire une bêta-lactamase ou une protéine hybride dérivée d'une bêta-lactamase.

Bien que les nouveaux mutants présentent, tout comme les mutants excréteurs de phosphatase alcaline, une mutation au voisinage du marqueur _gal_, certains d'entre eux présentent des propriétés différentes: en effet, ils ne sont pas sensibles aux inhibiteurs de croissance, ils sont sensibles à la colicine E1 et ils n'excrètent pas la phosphatase alcaline, ou en excrètent moins de 20% à 30°C.

L'obtention de ces nouveaux mutants montre donc qu'il est possible d'obtenir des mutants excréteurs qui sont capables d'excréter de façon sélective une protéine périplasmique ou un nombre limité de protéines périplasmiques.

Les nouveaux mutants de l'invention permettent l'obtention en quantité importante de la bêta-lactamase, sans qu'il soit nécessaire de procéder à une lyse cellulaire puisque cette protéine est excrétée par lesdits mutants dans le milieu de culture. On sait par ailleurs que la bêta-lactamase est une enzyme d'intérêt industriel. Les mutants, objets de la présente demande peuvent en conséquence être utilisés pour la production de la bêta-lactamase elle-même, ou encore comme hôtes bactériens pour des vecteurs recombinants plasmidiques portant des fusions entre tout ou partie du gène de structure de la bêta-lactamase (correspondant à la séquence amino-terminale de la protéine et contenant la séquence signal permettant l'exportation de la protéine dans l'espace périplasmique) et le gène de structure ou l'ADN complémentaire d'une protéine d'origine procaryotique, virale ou eucaryotique de façon à obtenir une protéine hybride qui sera excrétée dans le milieu extracellulaire. Cette propriété trouve de nombreuses applications en vue de la production et de la purification facilitée de protéines d'intérêt médical (hormones, vaccins) et industriel (enzymes) dont les gènes de structure ou les ADN complémentaires auront été convenablement clonés dans des véhicules plasmidiques porteurs du gène de structure d'une bêta-lactamase.

Les techniques de fusion de gènes in vitro ont été décrites par différents auteurs, et notamment par VILLA-KOMAROFF et al, (1978), Proc. Natl.Sci. USA, 75 : 3727 - 3731, dans le cas des fusions entre la bêta-lactamase et la proinsuline de rat.

Pour réaliser ce type de fusions, il convient d'utiliser un vecteur plasmidique portant le gène de structure de la bêta-lactamase. On opère par délétion partielle du gène de la bêta-lactamase, ouverture du plasmide au niveau du site de coupure, puis insertion et ligation du gène de structure de la protéine considérée.

La transformation des bactéries par ces vecteurs leur confère la capacité de synthétiser une protéine hybride possédant la séquence signal de la pro-bêta-lactamase, tout ou partie de la séquence polypeptidique de la bêta-lactamase et la séquence polypeptidique de la protéine choisie. Après transformation des mutants excréteurs de bêta-lactamase par les plasmides ainsi obtenus, il est possible d'identifier les souches qui synthétisent correctement la protéine hybride, l'exportent dans le périplasme où la séquence signal de la bêta-lactamase est éliminée, et qui l'excrétent dans le milieu extracellulaire.

Les méthodes et produits employés pour l'obtention et l'identification des nouveaux mutants objets de la présente demande vont maintenant être décrits ci-après.

BAD ORIGINAL

MATERIELS ET METHODES

1 - MATERIELS

1. SOUCHES BACTERIENNES

Toutes les souches utilisées dérivent d'Escherichia coli K-12. Leurs propriétés caractéristiques sont indiquées dans le tableau suivant:

| NOM | SEXE | GENOTYPE | ORIGINE |
|---|---|---|---|
| P4X | Hfr | metB1 | Référence 1 |
| Gal5 | Hfr | metB1, lacI | Mutant spontané de P4X synthétisant de façon constitutive la bêta-galactosidase |
| 188 | Hfr | metB1, lacI, phoS,T | Mutant spontané de Gal5 synthétisant de façon constitutive la bêta-galactosidase et la phosphatase alcaline |
| 188$^R$ | Hfr | metB1, lacI, phoS,T, amp$^R$, tet$^R$ | Dérivé de 188, transformé par le plasmide pBR322, résistant à l'ampicilline et à la tétracycline |
| 875 | Hfr | metB1, lacI, phoS,T, exc 875 amp$^R$, tet$^R$ | Mutants excréteurs de bêta-lactamase obtenus après mutagénèse de la souche |
| 880 | Hfr | metB1, lacI, phoS,T, exc 880 amp$^R$, tet$^R$ | 188$^R$ (objets de la présente demande de brevet) |
| 207$^R$ | Hfr | metB1, lacI, phoS,T, lky 207 amp$^R$, tet$^R$ | Mutants excréteurs de phosphatase alcaline (Référence 2) transformés par le plasmide pBR322 |
| 236$^R$ | Hfr | metB1, lacI, phoS,T, lky 236 amp$^R$, tet$^R$ | |

Référence 1 : JACOB F. et WOLLMAN E.L. (1961), Sexuality and the Genetics of

**0149936**

bacteria. Academic Press Inc., New York.

Référence 2 : J.C. LAZZARONI et R. PORTALIER (1979), FEMS Microbiology Letters 5, 411-416.

### 2. MILIEUX DE CULTURE

Milieu 63 : milieu synthétique : $KH_2PO_4$ (0,1M) ; $(NH_4)_2SO_4$ (15 mM) ; $MgSO_4,7H_2O$ (4 mM) ; $FeSO_4,7H_2O$ (9 μM) ; KOH (0,2 mM), pH 7,2.

Milieu BL : milieu riche, liquide, contenant : extrait de levure (5 g/1) bactotryptone (10 g/1) et chlorure de sodium (5 g/1), pH 7,3.

Les facteurs de croissance nécessaires sont ajoutés aux milieux synthétiques, aux concentrations suivantes : méthionine 0,4 g/1 ; proline, arginine, histidine, thréonine et leucine 0,1 g/1 ; adénine 0,03 g/1 ; thymine 0,05 g/1 ; vitamine B1 0,5 mg/1 ; glucose 4 g/1 (G).

### II - METHODES

#### 1. CULTURES BACTERIENNES

Les précultures sont effectuées en milieu riche BL.

La croissance bactérienne est estimée par la turbidité des suspensions, mesurée à 600 nm à l'aide d'un spectrophotomètre Jean et Constant. 100 unités Jean et Constant correspondent à une concentration cellulaire de $3.10^8$ bactéries par ml, soit 0,2 mg de poids sec bactérien par ml.

#### 2. METHODES BIOCHIMIQUES

##### 2.1. Dosages enzymatiques

Les activités enzymatiques sont déterminées selon les méthodes décrites par :

- GAREN et LEVINTHAL, (1960), Biochem. Biophys. Acta, 38 : 470-483;

- MILLER, (1972), Experiments in Molecular Genetics-Cold Spring Harbor Laboratory, Cold Spring Harbor New-York ;

- SYKES et NORDSTROM, (1972), Antim. Agents Chemother. 1 : 94-99, pour, respectivement, la phosphatase alcaline, la bêta-galactosidase et la bêta-lactamase.

Une unité enzymatique (U) correspond à la quantité d'enzyme qui hydrolyse :

- une nanomole de substrat par minute, dans le cas de la phosphatase alcaline et de la bêta-galactosidase (1U = 16,67 pkat) ;

- une micromole de substrat par minute, dans le cas de la bêta-lactamase (1U = 16,67 nkat).

##### 2.2. Techniques d'extraction enzymatique

a) Extraction à la presse de French

BAD ORIGINAL

Le contenu cellulaire des bactéries est libéré après éclatement des cellules sous l'action d'une pression mécanique élevée, à l'aide d'une presse de French. Les débris membranaires et les cellules non éclatées sont éliminés.

b) Choc Osmotique

La technique du choc osmotique permet, contrairement aux techniques précédemment décrites, de libérer sélectivement les enzymes périplasmiques, sans altérer la membrane cytoplasmique. La méthode utilisée est celle décrite par HEPPEL, op.cit. Les cellules en phase stationnaire de croissance, sont mises en suspension et agitées à température ambiante dans une solution hypertonique de saccharose (20 %) et d'EDTA ($10^{-3}$M), ce qui entraîne leur plasmolyse ainsi qu'une déstabilisation de la structure de leur membrane externe. Ces cellules sont ensuite transférées rapidement dans un milieu hypotonique froid (eau à + 3°C) : les composés périplasmiques sont alors libérés dans le milieu extérieur qui constitue le fluide osmotique ; celui-ci est ensuite séparé des cellules par centrifugation à 13000 g pendant 30 minutes.

2.3. Préparation d'extraits membranaires totaux

L'étude de la composition en protéines majoritaires de la membrane externe peut être effectuée sur des extraits d'enveloppe cellulaire totale, préparés selon une méthode dérivée des techniques déjà décrites (SCHNAITMAN, (1974), J. Bacteriol. 118 : 442-453 ; LUGTENBERG et al, (1975), FEBS Letters 58 : 254-258.).

Après culture jusqu'en phase stationnaire de croissance, les cellules sont centrifugées, lavées en tampon HEPES (10 mM) à pH 7,4, remises en suspension dans ce même tampon (à raison de $10^9$ à $5.10^9$ bactéries par ml), et désintégrées à la presse de French dans les conditions précédemment décrites (le tampon HEPES est une solution tampon commercialisée par la firme MERCK). Après élimination des cellules non éclatées par centrifugation 10 minutes à 6000 g, le surnageant est ultracentrifugé à 280000 g pendant 90 minutes. Le culot membranaire obtenu est lavé en tampon HEPES, ultracentrifugé une seconde fois puis remis en suspension dans de l'eau distillée (concentration finale correspondant à environ $10^{11}$ cellules par ml).

2.4. Electrophorèse sur gel de polyacrylamide

- Les gels de polyacrylamide sont préparés selon la technique décrite par LAEMMLI, (1970), Nature (London), 227 : 680-685, à une concentration de 12,5 % en acrylamide, ou selon un gradient d'acrylamide 7-17 %, dans le cas des analyses d'extraits membranaires.

2.5. Transformation des cellules bactériennes par de l'ADN plasmidique

BAD ORIGINAL

Le traitement des cellules d'E. coli par du chlorure de calcium "perméabilise" l'enveloppe cellulaire et permet la pénétration dans le cytoplasme de molécules d'ADN plasmidique. Les plasmides ainsi introduits demeurent dans les cellules hôtes, sont doués d'autoréplication et sont transmis à la descendance.

Les cellules d'E. coli ont été transformées par le plasmide pBR322 selon une méthode dérivée de celle décrite par LEDEBERG et COHEN, (1974), J. BACTERIOL. 119 : 1072-1074.

Le plasmide pBR322 et sa construction ont été décrits par F.BOLIVAR et al, Gene, (1977) 2, 75-93.

## 3. METHODES GENETIQUES

### 3.1. Tests de caractérisation in situ

a) Détection de l'activité et de l'excrétion de la phosphatase alcaline

L'activité phosphatase alcaline est estimée au niveau des colonies bactériennes par un test qualitatif (test in situ). Un mélange d'un volume égal d'alpha-naphtyl-phosphate (8mm) en Tris-HCL (1M) à pH 8,0 et d'o-dianisidine tétrazotée (20mM dans l'eau) est versé sur les colonies qui se sont développées sur milieu solide. L'alpha-naphtol libéré lors de l'hydrolyse de l'alpha-naphtyl-phosphate par la phosphatase alcaline forme, avec l'o-dianisidine tétrazotée, un précipité brun (MILLER, op. cit.). Les mutants excréteurs de l'enzyme sont caractérisés par la formation d'un large halo brun autour des colonies (LAZZARONI et PORTALIER, (1979), FEMS Microbiol. Letters, 5 : 411-416).

b) Détection de l'excrétion de la ribonucléase I

Après croissance des colonies bactériennes sur un milieu gélosé contenant de l'ARN de levure (SIGMA type IV à 15 g/1), une solution d'acide trichloroacétique froid à 10 % est versée afin de précipiter l'ARN non hydrolysé (WEIGAND et ROTHFIELD, (1976), J. Bacteriol. 125 : 340-345). Les colonies qui ont libéré la ribonucléase I dans le milieu extérieur, dégradant ainsi l'ARN en nucléotides, apparaissent entourées d'un halo translucide.

c) Détection de l'excrétion de la bêta-lactamase

Un test de détection de l'activité bêta-lactamase extracellulaire a été mis au point sur la base d'une méthode microbiologique.

Sur un milieu riche gélosé, contenant 20 microgrammes/ml d'ampicilline, on étale uniformément une suspension d'une souche sensible à l'antibiotique (P4X), à raison de $2.10^8$ cellules par boîte. La suspension cellulaire, transformée par pBR322, que l'on veut tester pour le caractère excréteur est étalée simultanément à raison de 200 à 300 cellules par boîte

BAD ORIGINAL

de Pétri.

Après 15 heures d'incubation à 37°C ou 20 heures à 30°C, les clones excréteurs de bêta-lactamase apparaissent entourés d'un halo de croissance de la souche P4X, qui correspond à la zone où l'antibiotique a été totalement hydrolysé par l'enzyme excrétée.

Il est possible d'adapter ce test pour l'analyse, par réplique, de clones déposés sous la forme d'un ensemencement circulaire important ("patches"). Dans ce cas, l'inoculum de la souche P4X, correspondant à $10^6$ cellules, est étalé sur un milieu contenant 100 microgrammes/ml d'ampicilline. Après absorption, les clones à tester sont répliqués sur ce milieu et incubés pendant 8 heures à 37°C ou 15 heures à 30°C.

Les conditions expérimentales de ce test ont été optimisées en fonction du milieu de culture, de la température, et des conditions de croissance (colonies isolées ou répliques de patches). Ces résultats sont résumés dans le tableau 2 suivant :

Tableau 2 : Test de caractérisation in situ de l'excrétion de bêta-lactamase. Conditions expérimentales optimales.

| CARACTERISTIQUES DU MILIEU INDICATEUR | CONDITIONS DE CROISSANCE | |
|---|---|---|
| | Test sur colonies isolées | Test sur répliques |
| Ampicilline (microgrammes/ml) | 20 | 100 |
| P4X (bact./ml) | $2.10^8$ | $10^6$ |

| Temps d'incubation (heures) Température (°C) | TEST SUR COLONIES ISOLEES | | TEST SUR REPLIQUES | |
|---|---|---|---|---|
| | Milieu riche | Milieu synthétique | Milieu riche | Milieu synthétique |
| 30 | 20 | 24-30 | 15 | 15 |
| 37 | 15 | 20-22 | 8 - 15 | 15 |
| 42 | 10 - 15 | 20-22 | 7 - 8 | 15 |

d) Sensibilité aux inhibiteurs de croissance

La sensibilité des mutants excréteurs de bêta-lactamase vis-à-vis de certains inhibiteurs de la croissance bactérienne, est estimée après culture en colonies sur un milieu riche gélosé contenant le composé toxique à une concentration qui permet le développement normal de la souche parentale : orange acridine (0,75 mM), EDTA (2 mM), mitomycine C (0,9 µM), chloramphénicol (0,6 µM), acide cholique (58 mM). Les clones qui ne se développent pas ou qui se multiplient plus lentement que la souche parentale sont considérés comme sensibles.

e) Résistance aux colicines El et A

La production des colicines El et A est réalisée à l'aide des souches colicinogéniques JF390 et PRC628, respectivement, selon la méthode décrite par SPUDICH et al, (1970), J. Mol. Biol. 53 : 49-67. Une concentration de 10 ml de suspension de colicine El ou A par litre de milieu de culture, est inhibitrice de la croissance des souches parentales.

f) Résistance aux bactériophages

La résistance des souches aux bactériophages est estimée par la méthode du "cross-streaking", qui consiste à déposer sur un milieu gélosé complet une goutte de lysat de phage sous la forme d'une strie, et une goutte de suspension de la souche à tester selon une autre strie qui croise la première de façon perpendiculaire. Le test est lu après une nuit d'incubation à 37°C. Le mutant est dit résistant si sa croissance n'est pas altérée après la rencontre du lysat, et sensible dans le cas contraire.

3.2. Transfert de matériel génétique

La préparation de lysats de bactériophages P1, les transductions généralisées par P1 ainsi que les croisements non interrompus, sont effectués selon les protocoles décrits (MILLER, op.cit.)

3.3. Mutagénèse et isolement des mutants excréteurs de bêta-lactamase

La mutagénèse de la souche parentale 188$^R$, porteuse du plasmide pBR322, est réalisée en présence de NTG, selon la méthode décrite par Miller, op.cit. Pour une concentration finale de 40 microgrammes/ml de NTG et un temps d'action de 20 minutes, le taux de survie de la souche est de 53 % et le taux de mutation pour le caractère de référence Lac⁻ est de 0,3 %.

Plusieurs cultures d'expression indépendantes de la suspension mutagénisée sont réalisées en milieu riche BL pendant 3 heures ; elles sont ensuite diluées et étalées à raison de 200 colonies par boîte de Pétri, sur le milieu indicateur de l'excrétion de bêta-lactamase, précédemment décrit, puis incubées à 30° ou 37°C pendant 15 ou 20 heures respectivement. Les clones identifiés comme excréteurs sont réisolés deux fois sur un milieu sélecteur du plasmide pBR322 (contenant 20 microgrammes/ml d'ampicilline) avant d'être analysés.

## RESULTATS

### 1 - ETUDES PHYSIOLOGIQUES

La souche 188[K], d'E.coli K-12, dérivée de l'Hfr P4X, et transformée par pBR322, synthétise de façon constitutive les enzymes bêta-galactosidase et phosphatase alcaline, respectivement localisées dans le cytoplasme et le périplasme.

Elle a été choisie comme souche parentale car elle présente l'avantage de posséder, quel que soit le milieu de culture utilisé, ces deux marqueurs enzymatiques de compartimentation, qui sont d'un emploi aisé.

### 1. Synthèse de la bêta-lactamase plasmidique chez la souche parentale

La souche parentale 188[R] a été cultivée en milieu riche BL, à 30°, 37° et 42°C, et les activités intra- et extra-cellulaire de la bêta-lactamase ont été mesurées.

Les résultats présentés dans le tableau suivant, montrent que la synthèse de bêta-lactamase est très dépendante de la température. L'activité spécifique mesurée après croissance de la souche 188[R] à basse température (30°C) est cinq fois plus importante que celle obtenue à 37°C. Lorsque la souche est cultivée à 42°C, cette activité diminue fortement et ne représente plus que 1 % de la valeur maximale mesurée à 30°C.

Le taux de bêta-lactamase excrétée dans le milieu de culture est minimum lorsque la souche est cultivée à 30°C. Il augmente de façon significative lorsque la température s'élève de 30° à 37°C (de 4 à 10 %), et peut atteindre 50 % de l'activité totale (0,2 unités), après croissance à 42°C. Ce phénomène est propre à la bêta-lactamase et ne s'étend ni à la phosphatase alcaline périplasmique, ni à la bêta-galactosidase cytoplasmique.

| | | | ACTIVITES ENZYMATIQUES | | | | | |
|:---|:---|:---|:---|:---|:---|:---|:---|:---|
| | TEMPERA- | | Bêta-lactamase | | Phosphatase | | Bêta-galac- | |
| MILIEU | TURE | | | | alcaline | | tosidase | |
| | (°C) | | A S* | A E ** | A E ** | | A E** | |
| | 30 | | 20 | 4 | 3 | | 3 | |
| BL | 37 | | 4 | 10 | 3 | | 2 | |
| | 42 | | 0,2 | 50 | 3 | | 2 | |

\* Activité spécifique intra + extra cellulaire, en unités par milligramme de poids sec bactérien (U/mg psb)

\*\* Activité extracellulaire (%)

## 2. Localisation périplasmique de la bêta-lactamase plasmidique

Afin de vérifier la compartimentation périplasmique de la bêta-lactamase codée par pBR322, on a soumis la souche parentale 188[R] à un choc osmotique, après culture en milieu riche EL à 37°C et récolté des cellules en phase stationnaire de croissance. L'activité bêta-lactamase a été dosée dans le surnageant de culture, dans les différentes fractions du choc osmotique (le stade I, constitué du surnageant recueilli après centrifugation des bactéries équilibrées en milieu hypertonique, et le fluide périplasmique proprement dit), et dans le cytoplasme après extraction à la presse de French.

On a estimé, en parallèle, les activités bêta-galactosidase et phosphatase alcaline dans ces différentes fractions, afin de vérifier que le choc osmotique a été réalisé dans de bonnes conditions et a permis une extraction sélective des protéines périplasmiques, sans libération des protéines cytoplasmiques.

Les résultats de ces travaux, présentés ci-dessous, confirment la localisation périplasmique de la bêta-lactamase plasmidique. En effet, plus de 90 % de cette activité sont retrouvés dans le fluide osmotique ; il en est de même pour le marqueur périplasmique de référence, la phosphatase alcaline.

Le taux de bêta-lactamase extracellulaire est inférieur à 4 % de l'activité totale.

La souche 188[R] synthétise la bêta-lactamase codée par le plasmide pBR322, l'exporte au niveau du périplasme mais ne l'excrète que faiblement dans le milieu extracellulaire. Elle satisfait donc aux critères requis pour être utilisée comme souche parentale, dans la recherche de mutants excréteurs de bêta-lactamase.

| ENZYMES | AS (U/mg psb) | REPARTITION DES ACTIVITES ENZYMATIQUES (%) | | | |
|---|---|---|---|---|---|
| | | Surnageant de culture | Stade 1 (Saccharose – EDTA) | Fluide périplas-mique | Culot cellu-laire résiduel cytoplasme |
| Bêta-lacta-mase | 22 | 3,8 | 1 | 93,7 | 1,5 |
| Phosphatase alcaline | 721 | 2,7 | 0,4 | 93,7 | 3,2 |
| Bêta-galac-tosidase | 5968 | 3 | 0,2 | 0,4 | 96,4 |

BAD ORIGINAL

II - ISOLEMENT ET CARACTERISATION PHYSIOLOGIQUE, GENETIQUE ET BIOCHIMIQUE
DE MUTANTS EXCRETEURS DE BETA-LACTAMASE

1. Isolement et caractérisation phénotypique de mutants excréteurs
de Bêta-lactamase

Après mutagénèse de la souche 188$^R$, induite par la NTG, le test in situ précédemment décrit nous a permis d'isoler à 30°, 37° et 42°C, une collection de mutants indépendants, excréteurs de bêta-lactamase.

Les différents mutants excréteurs de bêta-lactamase, ont tout d'abord été étudiés qualitativement. Les phénotypes examinés sont susceptibles de mettre en évidence des modifications de la structure de l'enveloppe cellulaire.

On a ainsi analysé :

- l'excrétion, dans le milieu extracellulaire, de diverses enzymes périplasmiques (phosphatase alcaline, ribonucléase I),

- l'incapacité à utiliser comme seule source de carbone, des sucres dont le catabolisme implique la présence de certaines protéines fixatrices localisées dans le périplasme (arabinose, galactose, maltose, ribose), ou de certaines perméases intégrées dans la membrane cytoplasmique,

- la sensibilité accrue envers certains inhibiteurs de la croissance bactérienne (agents chélatants, antibiotiques, sels biliaires),

- l'acquisition d'une plus grande résistance à l'égard de certains bactériophages, dont les récepteurs sont des constituants de la membrane externe.

- la tolérance envers certaines colicines.

Sur la base des tests qualitatifs permettant de mettre en évidence l'excrétion de la phosphatase alcaline (A), de la ribonucléase I (R) et de la bêta-lactamase (L), il a été possible d'identifier des souches qui sont capables d'excréter sélectivement la bêta-lactamase seule ou la bêta-lactamase et la ribonucléase I, sans excréter la phosphatase alcaline.

Par ailleurs, elles présentent des phénotypes différents des mutants excréteurs de phosphatase alcaline.

2. Caractérisation génétique des mutants excréteurs de bêta-lactamase

La plupart des mutations d'excrétion sont localisées près du marqueur gal, à la minute 16,5 du chromosome bactérien. Les taux de cotransduction de ces mutations avec le gène gal varient de 40 à 76 %.

Le tableau suivant résume les propriétés phénotypiques et génétiques de deux mutants excréteurs de bêta-lactamase de référence (875 et 880), comparativement aux mutants excréteurs de phosphatase alcaline (207 et 220).

PROPRIETES PHENOTYPIQUES ET GENETIQUES DES MUTANTS EXCRETEURS DE BETA-LACTAMASE

| PHENOTYPES TESTES | | SOUCHE PARENTALE 188$^R$ | MUTANTS EXCRETEURS DE BETA-LACTAMASE | | EXCRETEURS DE PHOSPHATASE | |
|---|---|---|---|---|---|---|
| | | | 875 | 880 | 207 | 236 |
| Excrétion de | L | + | E | E | E | E |
| protéines | R | + | E | E | E | E |
| périplasmiques | A | + | NE | NE | E | E |
| Inhibiteurs | AC | + | − | + | − | − |
| de | EDTA | + | + | + | − | − |
| croissance | CHL | + | + | + | − | − |
| Croissance | suc | + | + | + | + | + |
| sur | mal | + | +M | +M | + | + |
| sucres | gal | + | + | +M | + | + |
| | lac | + | + | + | + | + |
| | ara | + | + | + | +M | + |
| Bactério- | Tula | S | S | S | S/R | S |
| phages | Mel | S | S | S | S | S |
| | K3 | S | S | S | S | S |
| | T6 | S | S | S | S | S |
| Colicines | E1 | S | S | S | R | S |
| | A | S | S | R | R | R |
| Cotransduction de la mutation avec le marqueur gal | | / | 52 | 69 | 43 | 47 |

L, R, A : Bêta-lactamase, Ribonucléase I, Phosphatase Alcaline

AC, CHL : Acide cholique, Chloramphénicol

suc, mal, gal, lac, ara : succinate, maltose, galactose, lactate, arabinose

+ : croissance, − : non croissance, +M : croissance mucoïde

E : excrétion, NE : non excrétion

S : sensibilité, R : résistance

## 3. Analyse quantitative de l'excrétion de béta-lactamase

Afin d'évaluer l'excrétion de la béta-lactamase plasmidique dans le milieu extracellulaire, nous avons réalisé des cultures des mutants excréteurs, en milieu complet BL, à différentes températures (30 et 37°C).

Les activités enzymatiques béta-lactamase, phosphatase alcaline et béta-galactosidase ont été estimées dans les surnageants de culture et dans les extraits cellulaires totaux (périplasme et cytoplasme).

Le tableau suivant présente les résultats des dosages enzymatiques concernant les mutants 875 et 880, comparativement à la souche parentale $188^R$ et aux mutants excréteurs de phosphatase alcaline.

ANALYSE QUANTITATIVE DE L'EXCRETION DE LA BETA-LACTAMASE

| SOUCHES ANALYSEES | MILIEU (T°C) | BETA-LACTAMASE AS | AE | PHOSPHATASE AS | AE | BETA-GALACTOSIDASE AE |
|---|---|---|---|---|---|---|
| SOUCHE $188^R$ PARENTALE | BL 30 | 20 | 4 | 422 | 4 | 3 |
| | 37 | 4 | 10 | 539 | 3 | 2 |
| MUTANTS EXCRE- 880 TEURS DE BETA-LACTA- MASE 875 | BL 30 | 24 | 40 | 409 | 14 | 3 |
| | BL 37 | 14 | 96 | 432 | | 4 |
| | BL 30 | 18 | 28 | 479 | 18 | 2 |
| | 37 | 13 | 54 | 508 | 26 | 2 |
| MUTANT EXCRE- TEURS $(207^R)$ DE PHOSPHATASE | BL 30 | 19 | 77 | 465 | 85 | 5 |
| | BL 37 | 17 | 83 | 540 | 84 | 5 |

## 4. Caractérisation biochimique des mutants excréteurs de béta-lactamase

La composition protéique des différents compartiments intra- et extra-cellulaires des mutants excréteurs a été déterminée par analyse électrophorétique.

**0149936**

4.1. <u>Analyse de la composition protéique des surnageants de culture</u>

- Excrétion de plusieurs protéines périplasmiques majoritaires: la protéine 58 K, P1 (le monomère de la phosphatase alcaline), P2 (la protéine fixatrice du glycérol-3-phosphate), P4 (la protéine fixatrice du phosphate) et la bêta-lactamase.

- Excrétion de 5 protéines périplasmiques minoritaires, en quantités réduites.

- Excrétion d'une fraction des protéines majoritaires de la membrane externe : PhoE, OmpF, OmpC et OmpA.

4.2. <u>Analyse du contenu protéique des fluides périplasmiques</u>

Le contenu protéique de l'espace périplasmique de la plupart des mutants excréteurs analysés est globalement diminué par rapport à celui de la souche parentale $188^R$.

Cependant, une fraction non négligeable des espèces protéiques majoritaires excrétées (protéines 58K, P1, P2, P4 et bêta-lactamase) conserve une localisation périplasmique.

4.3. <u>Analyse de la composition protéique de la membrane externe de l'enveloppe cellulaire</u>

La majorité des mutants excréteurs analysés, et notamment les mutants 875 et 880, ne présentent pas d'importantes altérations du contenu protéique de leur membrane externe. On observe cependant une légère augmentation de la proportion relative de la protéine PhoE, dans le cas du mutant 880, et une diminution de la quantité de protéine OmpF, dans le cas du mutant 875, par rapport à la teneur totale en protéines majoritaires (PhoE, OmpF, OmpC, OmpA).

La souche parente ainsi que deux mutants excréteurs selon l'invention ont été déposés à la collection nationale de l'Institut PASTEUR le 23 novembre 1983 sous les numéros de dépôt suivants : $188^R$ : N°I-264 ; 875 : N°I-265 ; 880 : N°I-266.

La présente invention a donc pour objet de nouveaux mutants d'<u>Escherichia coli</u>, capables d'excréter dans le milieu extracellulaire des protéines périplasmiques, caractérisés par le fait qu'ils sont issus d'une souche parente capable de synthétiser une bêta-lactamase ou une protéine hybride comportant une séquence amino-terminale analogue à celle de la bêta-lactamase, ladite souche parente n'excrétant pas les protéines périplasmiques y compris la bêta-lactamase ou ladite protéine hybride, ou n'en excrétant pas plus de 5 % à 30°C, que lesdits mutants sont porteurs d'une mutation cotransductible à plus de 50 % avec le marqueur <u>gal</u>, qu'ils sont, comme la souche parente, non sensibles ou peu sensibles à un ou plusieurs des inhibiteurs de croissance choisis parmi l'acide cholique, le chloramphénicol et l'EDTA, qu'ils sont sensibles, comme la souche parente, à la colicine El, que, comme la souche parente, ils n'excrétent pas la phosphatase alcaline ou n'en excrétent pas plus de 20 % à 30°C, et qu'ils excrètent au moins 50 % de la bêta-lactamase ou de ladite protéine hybride qu'ils produisent à 37°C.

Les nouveaux mutants de l'invention peuvent présenter notamment les caractéristiques suivantes prises isolément ou en combinaison :

1 - ils donnent des clones réverses spontanés non excréteurs de la bêta-lactamase avec une fréquence de réversion de $10^{-7}$ à $10^{-8}$ ;

2 - ils sont sensibles à l'un au moins des bactériophages suivants: TuIa, MeI, K3 et T6 ;

3 - ils sont sensibles à l'une au moins des deux colicines El et A;

4 - ils sont résistants à l'une au moins des drogues suivantes : acide cholique, EDTA, chloramphénicol ;

5 - la composition protéique de leur membrane présente, par comparaison avec la souche parente, soit une légère augmentation de la proportion relative de la protéine PhoE, soit une légère diminution de la quantité de protéine OmpF, par rapport à la teneur totale en protéines majoritaires PhoE, OmpF, OmpC et OmpA ;

6 - ladite souche parente est une souche modifiée par un plasmide portant le gène de structure d'une bêta-lactamase ; ledit plasmide est par exemple le plasmide pBR322 (BOLIVAR et al, (1977), Gene 2 : 75-93), ou tout autre plasmide de clonage analogue, ou encore un facteur de résistance aux antibiotiques (facteur R) ;

7 - la souche parente (notée 188$^R$) est la souche 188 modifiée par ledit plasmide.

Parmi les mutants excréteurs suivant l'invention, on peut citer notamment ceux qui sont obtenus par mutagénèse, et en particulier les mutants 880 et 875 dont les propriétés ont été décrites précédemment.

L'invention a également pour objet un procédé d'obtention des mutants excréteurs de bêta-lactamase tels que définis précédemment. Ce procédé est caractérisé par le fait que l'on choisit une souche parente n'excrétant pas les enzymes périplasmiques ou n'en excrétant pas plus de 5 % à 30°C, ladite souche parente étant résistante à l'acide cholique, au chloramphénicol et à l'EDTA, que l'on soumet ladite souche parente à l'action d'un agent mutagène et que l'on identifie les mutants excrétant au moins 50 % de la bêta-lactamase ou de ladite protéine hybride qu'ils produisent à 37°C et n'excrétant pas la phosphatase alcaline ou n'en excrétant pas plus de 20 % à 30°C.

0149936

Dans ses modes d'exécution, le procédé de l'invention peut encore présenter les caractéristiques suivantes prises isolément ou en combinaison :

- on identifie les clones excréteurs de bêta-lactamase par croissance sur un milieu contenant un antibiotique à cycle bêta-lactame en présence d'une souche indicatrice sensible audit antibiotique et les clones excrétant la bêta-lactamase apparaissent alors entourés d'un halo de croissance de la souche indicatrice.

- Parmi ces clones, on écarte ceux excrétant d'importantes quantités de phosphatase alcaline et donnant par conséquence une réponse positive à un test coloré spécifique de cette enzyme. Comme indiqué ci-dessus, on peut conserver les clones n'excrétant pas plus de 20 % de la phosphatase alcaline qu'ils produisent, et donnant par conséquent une faible réponse au test coloré.

La présente invention a également pour objet, comme cela était précisé ci-dessus, l'application des nouveaux mutants excréteurs à la production d'une bêta-lactamase ou d'une protéine hybride dérivée d'une bêta-lactamase par un procédé qui se réalise en l'absence de lyse bactérienne. Ce procédé est caractérisé par le fait que l'on cultive dans un milieu convenable lesdits mutants excréteurs ou lesdits mutants excréteurs modifiés par un vecteur plasmidique porteur d'une fusion entre le gène de structure de la bêta-lactamase et le gène de structure ou un ADN complémentaire capable de coder pour une protéine que l'on désire faire synthétiser, et que l'on recueille dans le milieu extracellulaire, soit ladite bêta-lactamase, soit ladite protéine hybride.

**0149936**

1. Mutants d'Escherichia coli, capables d'excréter dans le milieu extracellulaire des protéines périplasmiques, caractérisés par le fait qu'ils sont issus d'une souche parente capable de synthétiser une bêta-lactamase ou une protéine hybride comportant une séquence amino-terminale analogue à celle de la bêta-lactamase, ladite souche parente n'excrétant pas les protéines périplasmiques y compris la bêta-lactamase ou ladite protéine hybride, ou n'en excrétant pas plus de 5 % à 30°C, que lesdits mutants sont porteurs d'une mutation cotransductible à plus de 50 % avec le marqueur gal, qu'ils sont, comme la souche parente, non sensibles ou peu sensibles à un ou plusieurs des inhibiteurs de croissance choisis parmi l'acide cholique, le chloramphénicol et l'EDTA, qu'ils sont sensibles, comme la souche parente, à la colicine EI, que, comme la souche parente, ils n'excrètent pas la phosphatase alcaline ou n'en excrètent pas plus de 20 % à 30°C, et qu'ils excrètent au moins 50 % de la bêta-lactamase (ou de ladite protéine hybride) qu'ils produisent à 37°C.

2. Mutants selon la revendication 1, caractérisés par le fait que ladite souche parente est une souche modifiée par un plasmide portant le gène de structure d'une bêta-lactamase.

3. Mutants selon la revendication 1, caractérisés par le fait qu'ils donnent des clones réverses spontanés non excréteurs de la bêta-lactamase avec une fréquence de réversion de $10^{-7}$ à $10^{-8}$.

4. Mutants selon l'une quelconque des revendications précédentes caractérisés par le fait qu'ils sont sensibles à au moins un bactériophage choisi parmi les bactériophages suivants : TuIa, MeI, K3 et T6.

5. Mutants selon la revendication 2, caractérisés par le fait que ledit plasmide est le plasmide pBR322 ou tout autre plasmide de clonage analogue ou encore un facteur R.

6. Mutants selon l'une quelconque des revendications 2 à 5, caractérisés par le fait que la souche parente est une souche modifiée par le plasmide pBR322, qu'ils sont sensibles à l'une au moins des deux colicines EI et A, qu'ils sont résistants à l'une au moins des drogues suivantes : acide cholique, EDTA et chloramphénicol, et qu'ils sont sensibles à l'un au moins des bactériophages suivants :TuIa, MeI, K3 et T6.

7. Mutants selon l'une quelconque des revendications 2 et 5, caractérisés par le fait que la souche parente est la souche déposée à la collection nationale de l'Institut Pasteur sous le n° I-264.

8. Mutant excréteur selon la revendication 7, caractérisé par le fait qu'il s'agit du mutant déposé à la collection nationale de l'Institut Pasteur sous le n° I-265.

9. Mutant excréteur selon la revendication 7, caractérisé par le fait qu'il s'agit du mutant déposé à la collection nationale de l'Institut Pasteur sous le n° 1-266.

10. Procédé d'obtention de mutants tels que définis dans l'une quelconque des revendications précédentes, caractérisé par le fait que l'on choisit une souche parente n'excrétant pas les enzymes périplasmiques ou n'en excrétant pas plus de 5 % à 30°C, ladite souche parente étant résistante à l'acide cholique, au chloramphénicol et à l'EDTA, que l'on soumet ladite souche parente à l'action d'un agent mutagène et que l'on identifie les mutants excrétant au moins 50 % de la bêta-lactamase ou de ladite protéine hybride qu'ils produisent à 37°C et n'excrétant pas la phosphatase alcaline ou n'en excrétant pas plus de 20% à 30°C.

11. Procédé selon la revendication 10, caractérisé par le fait que l'on identifie les clones excréteurs de bêta-lactamase par croissance sur un milieu contenant un antibiotique à cycle bêta-lactame en présence d'une souche indicatrice sensible audit antibiotique.

12. Procédé selon l'une quelconque des revendications 10 et 11, caractérisé par le fait que l'on écarte les clones excréteurs de phosphatase alcaline donnant une réponse positive à un test coloré spécifique de cette enzyme.

13. Application des mutants selon l'une quelconque des revendications 1 à 9 , à la production d'une bêta-lactamase ou d'une protéine hybride dérivée d'une bêta-lactamase, sans lyse bactérienne, caractérisée par le fait que l'on cultive dans un milieu convenable lesdits mutants excréteurs ou lesdits mutants excréteurs modifiés par un vecteur plasmidique porteur de fusion entre le gène de structure de la bêta-lactamase et le gène de structure ou un ADN complémentaire capable de coder pour une protéine que l'on désire faire synthétiser, et que l'on recueille dans le milieu extracellulaire, soit la bêta-lactamase, soit ladite protéine hybride.

BAD ORIGINAL